(19) Europäisches Patentamt
European Patent Office
Office européen des brevets

(11) Veröffentlichungsnummer: **0 446 852 A2**

(12) # EUROPÄISCHE PATENTANMELDUNG

(21) Anmeldenummer: **91103722.4**

(22) Anmeldetag: **12.03.91**

(51) Int. Cl.[5]: **C08L 67/04**, C08L 101/00, //(C08L67/04,67:04), (C08L101/00,67:04)

(30) Priorität: **13.03.90 DE 4007882**

(43) Veröffentlichungstag der Anmeldung: **18.09.91 Patentblatt 91/38**

(84) Benannte Vertragsstaaten: **AT BE CH DE DK ES FR GB GR IT LI LU NL SE**

(71) Anmelder: **BOEHRINGER INGELHEIM KG**
**W-6507 Ingelheim am Rhein(DE)**
(84) **AT BE CH DE DK ES FR GB GR IT LI LU NL SE**

Anmelder: **BOEHRINGER INGELHEIM INTERNATIONAL G.M.B.H.**
**W-6507 Ingelheim am Rhein(DE)**
(84) **GB**

(72) Erfinder: **Entenmann, Günther, Dr. Dipl.-Chem.**
**Schützenpfad 16**
**W-6507 Ingelheim am Rhein(DE)**
Erfinder: **Bielzer, Raffael**
**Kiebitzweg 7**
**W-5030 Hürth(DE)**
Erfinder: **Offergeld, Heinz, Dr.**
**Hirschgraben 9-11**
**W-5100 Aachen(DE)**

(54) **Verwendung von Polyglycolsäure und ihrer Derivate als Nukleierungsmittel.**

(57) Die Erfindung betrifft die Verwendung von Polyglycolsäure und ihrer Derivate als Nukleierungsmittel.

EP 0 446 852 A2

Die Erfindung betrifft die Verwendung von Polyglycolsäure und ihrer Derivate als Nucleierungsmittel bei der Kristallisation von Polymeren insbesondere von Polylactiden.

Polylactide weisen - als vom menschlichen oder tierischen Organismus abbaubare (resorbierbare) Kunststoffe - eine Vielzahl von Anwendungsmöglichkeiten auf. Dabei steht neben der Verwendung derartiger Polyester als Nahtmaterial ebenso der Einsatz in Form von festen Formkörpern - wie z.B. Schienen, Schrauben und Stiften, die als Ersatz für die entsprechenden Gegenstände aus Metalle dienen - im Vordergrund des Interesses. Damit diese Formkörper jedoch möglichst alle mechanischen Stabilitätskriterien in vergleichbarem Maße erfüllen, wie die entsprechenden Gegenstände aus Metall, werden hohe Anforderungen an den Kristallisationsgrad des jeweiligen Polylactids gestellt.

Der Kristallisationsgrad des bei der Herstellung des Implantates verwendeten Kunststoffes beeinflußt nicht nur die mechanischen Eigenschaften, sondern auch das Abbauverhalten des Formkörpers im menschlichen bzw. tierischen Organismus.

Weiterhin besteht die Gefahr, daß sich z.B. Prothesen, die keine oder nur geringe Kristallinität aufweisen, sich beim Erwärmen - z.B. bei der Sterilisation im Heißluftstrom - im Rahmen der sog. Nachkristallisation speziell in den Randbereichen in einem derartigen Ausmaß verziehen, daß das Implantat für den ursprünglich beabsichtigten Einsatz unbrauchbar wird. Bei derartigen Produkten kann auch bei der Lagerung oder sogar nach der Implantation in den Organismus Nachkristallisation und dadurch eine zeitabhängige Veränderung der Eigenschaften auftreten.

Um bei der Verarbeitung - beispielsweise - von Poly-L-lactid für bioabbaubare Implantate im Spritzgießverfahren hochkristalline Formteile zu erhalten, waren bisher lange Kühlzeiten erforderlich.

Ein Abkühlungsprozess mit derartig langen Verweilzeiten des bzw. der jeweiligen Formkörper(s) in der Spritzgießform, ist jedoch mit einem molekularen Abbau des eingesetzten Polylactids verbunden, welcher neben einer Änderung des Abbauverhaltens im Körper auch mit einer Verminderung der mechanischen Stabilitätseigenschaften verbunden ist.

Durch den Einsatz von sogenannten Nukleierungsmitteln konnte jedoch eine Möglichkeit eröffnet werden, hochkristalline Formkörper - auch bei Anwendung kürzerer Kühlzeiten - zu erhalten.

Bei der Nukleierung werden der Kunststoffschmelze Nukleierungsmittel zugefügt, die dort sog. heterogene Keime bilden. Durch die Zugabe derartiger Substanzen wird somit bereits vor der Bildung sog. "thermischer Kristallisationskeime" eine hohe Keimdichte erzielt, womit die Kristallisation früher eingeleitet und die Kristallisationszeit selbst verkürzt wird.

Aufgrund der Tatsache, daß bei der Temperatur, die für das Kristallwachstum am günstigsten ist, zahlreiche Kristallkeime in der Schmelze zugegen sind, wird das geschmolzene Material bei der Kristallisation entsprechend schnell verbraucht, woraus in dem nukleierten Material eine Gefügestruktur resultiert, die wesentlich feiner ist, als diejenige des entsprechenden nicht-nukleierten Materials.

Da jedoch die Zusammenhänge zwischen der Art des Kunststoffes und der chemischen und physikalischen Struktur des Keimbildners und der Nukleierung selbst noch ungeklärt sind, existieren bislang keine allgemeingültigen Regeln zur Auswahl des Nukleierungsmittels. Die meisten Keimbildner müssen daher auf empirischer Basis ermittelt werden. Die wichtigsten Anforderungen sind:

a) Der Keimbildner soll durch das Polymer benetzt oder absorbiert werden.

b) Der Keimbildner soll im Polymer unlöslich sein.

c) Der Schmelzpunkt des Keimbildners soll höher liegen als derjenige des Polymeren.

d) Der Keimbildner soll in möglichst feiner Form (1 bis 10 $\mu$m) in der Polymerschmelze dispergierbar sein.

e) Der Keimbildner soll eine möglichst geringe Oberflächenenergie besitzen.

Durch den Einsatz eines Keimbildners setzt die Kristallisation - im Vergleich zu einer Probe ohne Keimbildner - bei einer höheren Temperatur ein. Die Höhe der Kristallisationstemperatur - welche z.B. durch DSC-Untersuchungen (Differential-Scanning-Calorimetrie) leicht ermittelt werden kann - ist dabei ein Maß für die Wirksamkeit des Nukleierungsmittels. Allgemein gilt: Je größer die Differenz zwischen der Kristallisationstemperatur mit und ohne Keimbildnerzugabe ist, desto wirkungsvoller ist das Nukleierungsmittel.

Da die meisten Keimbildner bei der Verarbeitung zu höheren Kristallinitätsgraden führen, wird in den meisten Fällen auch die Ausbildung feinsphärolitischer Strukturen ermöglicht.

Dadurch bedingt werden im Vergleich zu dem entsprechend nicht-nukleiertem Material die Härte, der E-Modul (Elastizitätsmodul), die Zugfestigkeit, die Reißdehnung und die Schlagzähigkeit verbessert.

Besondere Bedeutung kommt dabei der Verkürzung der Zykluszeit beim Spritzgießen nukleierter Formassen zu. Aufgrund der Tatsache, daß die Kristallisation schon bei höheren Temperaturen einsetzt und demgemäß - beim Abkühlen - auch früher beendet ist, kann die sogenannte Entformungstemperatur heraufgesetzt und somit die Kühlzeit reduziert werden, woraus sich die angestrebte Zykluszeitverkürzung

ergibt.

Es ist daher die Aufgabe der vorliegenden Erfindung, ein Nukleierungsmittel zur Verfügung zu stellen, welches die Kristallisation von Poly-L-lactid bei höheren Temperaturen ermöglicht und das somit bei der Verarbeitung von Poly-L-lactid im Spritzgießverfahren eine entsprechende Heraufsetzung der Entformungstemperatur und auf diesem Wege eine Zykluszeitverkürzung erlaubt.

Es ist eine weitere Aufgabe der vorliegenden Erfindung, ein Nukleierungsmittel zur Verfügung zu stellen, welches biologisch abbaubar ist.

Es ist eine weitere Aufgabe der vorliegenden Erfindung ein Nukleierungsmittel zur Verfügung zu stellen, das ferner körperverträglich ist d.h. keine Abwehrreaktionen im Körper induziert und welches nicht toxisch ist.

Es wurde nun überraschenderweise gefunden, daß sich Polyglycolsäure und ihre Derivate der allgemeinen Formel I -

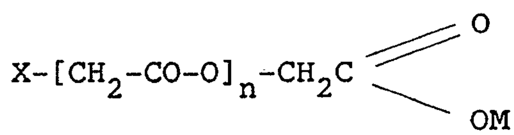

(I)

worin

X Wasserstoff, Halogen oder eine Hydroxylgruppe und

M ein Metall-Nation oder Wasserstoff

bedeutet -

als Nukleierungsmittel bei der Kristallisation - z.B. von Poly-L-lactid - geeignet sind.

Halogen bedeutet neben Fluor, Brom und Jod in erster Linie Chlor.

M bedeutet neben Lithium und Kalium in erster Linie Natrium.

Anstelle eines Erdalkalicarboxylates kann aber auch ein Carbonsäureester als Carboxylendgruppe Verwendung finden. Als Beispiel seien Alkylester genannt.

Polyglycolsäuren der Formel I können in an sich bekannter Weise durch Kondensation von Glycolsäure unter Wasserabspaltung, durch ringöffnende Polymerisation von Glycolid oder durch Polymerisation von Salzen von Halogenessigsäuren unter Abspaltung von Halogeniden erhalten werden. Stellt man die Polyglycolsäure z.B. durch Polymerisation von Natriumchloracetat her, so erhält man unter Abspaltung von Natriumchlorid ein Produkt der Formel I mit X = Cl und M = Na. Bei der anschließenden Abtrennung des Natriumchlorids durch Auswaschen mit Wasser oder in einem gesonderten nachfolgenden Reaktionsschritt kann je nach den Reaktionsbedingungen das Halogen ganz oder teilweise durch eine Hydroxygruppe, das Metall-Kation ganz oder teilweise durch ein Proton ersetzt werden. Je nach Gründlichkeit und Dauer des Auswaschprozesses kann das Produkt noch unterschiedliche Mengen an ionogenem Chlorid enthalten. So wurden in einer Reihe von Ansätzen aus Natriumchloracetat Produkte der Formel I mit folgenden Halogengehalten hergestellt:

| ionogenes Chlor | kovalent gebundendes Chlor |
|---|---|
| 0,33 % | 0,58 % |
| 0,01 % | 0,62 % |
| 0,03 % | 0,41 % |
| 0,02 % | 0,64 % |
| 0,10 % | 0,20 % |

Der Gehalt an ionogenem Chlor ist nicht kritisch. Er kann zwischen 0 und 5 % betragen. Bevorzugt

werden jedoch Produkte mit weniger als 1 % ionogenem Chlor. Auch der Gehalt an kovalent gebundenem Chlor ist an sich nicht kritisch. Da er jedoch ein Maß für das Molekulargewicht der Polyglycolsäure darstellt und dieses zur Erzielung eines ausreichend hohen Schmelzpunktes bestimmte Werte nicht unterschreiten sollte, beträgt der Gehalt an kovalent gebundenem Chlor zweckmäßigerweise zwischen 0 und 2 %. Dies schließt nicht aus, daß für bestimmte Anwendungszwecke ein Produkt mit höherem Chlorgehalt verwendbar ist, bevorzugt werden jedoch Produkte mit weniger als 1 %. Besonders bevorzugt ist ein Gehalt an kovalent gebundenem Chlor zwischen 0 und 0,6 %.

Der Gehalt an Natrium setzt sich aus dem nicht ausgewaschenen Natriumchlorid und dem gemäß Formel I an das Polymer gebundenen Anteil zusammen. Er ist nicht kritisch und kann je nach Produktqualität 5 % oder mehr betragen. Bevorzugt werden jedoch Produkte mit Natriumgehalten zwischen 0 und 2 %, besonders bevorzugt zwischen 0 und 1 %.

Die Zahl n in Formel I ist an sich nicht kritisch. Reaktionen wie sie für die Herstellung erfindungsgemäßer Polyglykolsäure angewandt werden, liefern jedoch aufgrund der Reaktionskinetik Produkte mit n von etwa 20 bis etwa 300. Die Herstellung noch höhermolekularer Produkte erfordert sehr lange Reaktionszeiten oder eine aufwendige ringöffnende Polymerisation von Glycolid. Für das erfindungsgemäße Verfahren sind jedoch so hohe Molekulargewichte nicht erforderlich. Bevorzugt werden daher Produkte, bei denen n zwischen 20 und 300, besonders bevorzugt zwischen 50 und 150 liegt.

Die Beschaffenheit der erfindungsgemäßen Produkte der Formel I wurde mit X = Chlor und M = Natrium erläutert. Die Erfindung ist jedoch nicht darauf beschränkt. X kann erfindungsgemäß auch ein anderes Halogen, Wasserstoff, eine Hydroxylgruppe oder eine andere Gruppe bedeuten, soweit sie nicht den Schmelzpunkt in unvorteilhafter Weise absenkt oder die Toxizität erhöht. Gleiches gilt auch für M, das auch - wie erläutert - Wasserstoff oder ein anderes Metall-Kation sein kann. Auch eine veresterte Carboxylendgruppe ist möglich, soweit dadurch nicht der Schmelzpunkt abgesenkt wird. Damit wird deutlich, daß der zu Produkten der Formel I führende Syntheseweg nicht kritisch ist.

Polymere der Glycolsäure sind, wie vielfach bewiesen wurde (z.B. durch ihre Verwendung als chriurgisches Nahtmaterial) gut verträglich und im Organismus abbaubar. Dies macht die Verbindungen der Formel I besonders geeignet zur Nukleierung von biologisch abbaubaren Polymeren auf der Basis von L-Lactid, D,L-Lactid, meso-Lactid, Glycolid, Trimethylencarbonat, Dioxanon, Caprolacton usw., soweit die Polymeren bzw. Copolymeren aufgrund ihrer Zusammensetzung kristallin sein können. Gleiches gilt auch für Mischungen (Blends) aus solchen Polymeren und Copolymeren. Damit ist jedoch keine Einschränkung der Erfindung verbunden. Verbindungen der Formel I können vorteilhaft auch zur Nukleierung nicht biologisch abbaubarer Kunststoffe eingesetzt werden, soweit die Verarbeitungstemperaturen unter der Erweichungstemperatur von I (ca. 200-230 für X=Cl und M=Na) liegt. Beispielhaft seien Polyethylen, Polypropylen, Polybutylen, Polyoxymethylen und Polyamid genannt.

Das erfindungsgemäße Nukleierungsmittel kann in einem Bereich von 0,2 bis 5 Gew.-%, vorzugsweise in einem Bereich von 0,4 bis 3 Gew.-% und ganz besonders bevorzugt in einem Bereich von 0,5 bis 2 Gew-% dem jeweiligen Polymeren - im vorliegenden Fall Poly-L-lactid - zugefügt werden. In Abhängigkeit von dem zu nukleierenden Polymeren kann es jedoch auch notwendig sein, von diesen Prozentsätzen abzuweichen und das erfindungsgemäße Nukleierungsmittel in großen oder kleinen Anteilen zuzufügen.

## Beispiele

Zur Herstellung von Proben für die DSC-Versuche wurden folgende Verfahrensschritte durchgeführt:

1. Herstellen von Mischungen (jeweils 50 g) aus Rohmaterial mit verschiedenen Gewichtsanteilen Nukleierungsmittel.
2. Extrusion der Mischungen in einem handelsüblichen Schweißextruder.

### Differentialkalorimetrie-Untersuchungen

Für die DSC-Untersuchungen wurden jeweils 3 Versuche mit dem Material einer Probe gemacht. Dabei wurde das Analysenmaterial aus dem letztem Drittel des extrudierten Stranges verwendet. Die Messungen wurden nach folgendem Schema durchgeführt:

- Einwaage einer Probe von ca. 10 mg
- Durchlaufen des folgenden Temperaturprofils:
  1) Aufheizen von Raumtemperatur mit einer Aufheizrate von 40 K/min auf eine Temperatur von 190 °C.
  2) Isotherme Temperaturführung, um die Schmelze zu homogenisieren.
  3) Abkühlen der Probe mit einer Abkühlrate von 10 K/min auf Raumtemperatur.

Fig. 1 zeigt in graphischer Darstellung den Anstieg der Kristallisationstemperatur in Abhängigkeit vom jeweiligen Anteil an Nukleierungsmittel.

Fig. 2 zeigt in graphischer Darstellung die Abhängigkeit der Kristallisationswärme vom jeweiligen Anteil an Nukleierungsmittel.

Diese Ergebnisse belegen, daß mit dem erfindungsgemäßen Nukleierungsmittel unter den angewandten Versuchsbedingungen eine maximale Steigerung der Kristallisationstemperatur von 100 °C auf 116 °C und eine Zunahme der Kristallisationswärme von 5 J/g auf 41 J/g bei steigendem Nukleierungsmittelanteil beobachtet werden kann. Oberhalb eines Anteils von 1,5 Gew.-% an Nukleierungsmittel ist keine merkliche Steigerung der Kristallisationstemperatur sowie der Kristallisationswärme festzustellen.

Spritzgießversuche

Für die Spritzgießversuche wurde eine Mischung aus Rohmaterial und Nukleierungsmittel mit einem Nukleierungsmittelanteil von 1,5 Gew.-% hergestellt.

Am Spritzgießwerkzeug wurde eine Wandtemperatur von 116 °C eingestellt.

Die Durchführung der Spritzgießversuche wurde mit einer Micro-Prazisionsspritzgießmaschine mit Kolbeneinspritzung durchgeführt, wobei der Kolben selbst in der Plastifizierschnecke geführt wird.

Aufgrund des Umstandes, daß der Feuchtigkeitsgehalt des eingesetzten Versuchsmaterials einen erheblichen Einfluß auf den Molekulargewichtsabbau in der Schmelze hat, ist es notwendig, vorgetrocknetes Material in das Spritzgießverfahren einzuführen.

Für das Trocknen wurde ein Absorptionstrockner verwandt, der mit dem auf der Spritzgießmaschine befindlichen Trichter verbunden war. Im Umluftverfahren wurde dabei dem Trichterinhalt von unten erwärmte und getrocknete Luft aus dem Absorptionstrockner zugeführt.

Das eingesetzte Poly-L-lactid-Rohmaterial wurde zunächst bei 140 °C für einen Zeitraum von 4 h getrocknet und anschließend mit 1.5 Gew.-% des Nukleierungsmittels vermischt. Im darauffolgenden Preßvorgang wurden Platten hergestellt, die mit einer Schneidmühle granuliert werden konnten.

Zur Vermeidung eines Molekulargewichtsabbaus erfolgte die Herstellung bei möglichst niedriger Temperatur und geringer Zykluszeit. Dabei wurde folgende Vorgehensweise angewandt:

1. Aufheizen des Materials unter geringem Druck bei einer Temperatur von 170 °C über einen Zeitraum von 1 min.
2. Verdichten der vorgewärmten Masse für einen Zeitraum von 10 s bei maximalem Druck.
3. Schnelles Abkühlen der Platten mittels Preßluft.

Durch Regranulieren des resultierenden Materials auf einer Schneidmühle wurde ein gut verarbeitbares Granulat hergestellt, welches unmittelbar vor dem Spritzgießen einer erneuten vierstündigen Trocknung bei 140 °C unterworfen wurde.

Formteilanalyse

Zur Beschreibung der Änderungen der Formteileigenschaften durch die Verwendung des erfindungsgemäßen Nukleierungsmittels, wurden an den im Spritzgießverfahren hergestellten Formteilen folgende Untersuchungen durchgeführt:

1. Thermoanalytische Untersuchungen

Für die thermoanalytische Untersuchung zur Bestimmung des Kristallisationsgrades wurde ebenfalls die DSC-Analyse angewandt, wobei eine Probe von ca. 7 mg Masse - von Raumtemperatur ausgehend - mit einer Aufheizrate von 20 K/min auf eine Temperatur von 200 °C aufgeheizt wurde.

Fig. 3 gibt die graphische Darstellung einer DSC-Analyse von einer Probe wieder, die ohne Nukleierungsmittel gespritzt wurde (Werkzeugtemperatur: 107 °C, Kühlzeit 60 s). Dabei führt die unvollständige Kristallisation während des Spritzgießvorganges zu einem exothermen Peak. Die bei der Nachkristallisation - bei einer Temperatur von 103 °C - freiwerdende Wärmemenge ($H_{nach}$) beträgt ca. 30 J/g. Die Schmelztemperatur liegt bei ca. 179 °C und die zum Aufschmelzen der kristallinen Bereiche benötigte Schmelzwärme ($H_{sc}$) beträgt 51 J/g.

Mit folgender Gleichung

$$X = \frac{H_{sc} - H_{nach}}{H_c}$$

worin

$H_{nach}$ die Nachkristallisationswärme

$H_{sc}$ die Schmelzwärme der Probe und

$H_c$ die Schmelzwärme einer 100%-ig kristallinen Probe von Poly-L-lactid ($H_c$ = 96,3 J/g)

bedeutet, läßt sich der Kristallisationsgrad berechnen.

Fig. 4 zeigt die graphische Darstellung des Kristallisationsgrades in Abhängigkeit von der Kühlzeit jeweils von Proben mit und ohne Nukleierungsmittel.

Aus Fig. 4 ist zu entnehmen, daß der Kristallisationsgrad einer nukleierten Probe über dem gesamten Kühlzeitbereich höher ist als derjenige der nicht-nukleierten Probe. Des weiteren ist den - in Fig. 4 wiedergegebenen - experimentellen Daten zu entnehmen, daß die Differenz des Kristallisationsgrades zwischen nukleiertem und nicht-nukleiertem Material mit kürzer werdender Kühlzeit zunimmt.

Die Durchführung einer Temperaturvariation des Werkzeuges (115 °C, 95 °C) bei der Herstellung der Formteile ohne das erfindungsgemäße Nukleierungsmittel führt zu keiner Erhöhung des Kristallisationsgrades gegenüber der Grundeinstellung von 107 °C.

Bei den Versuchen ohne Nukleierungsmittel kommt es bei einer Kühlzeitverkürzung von 120 s auf 60 s zu einem starken Abfall des Kristallisationsgrades von 50,4 % auf 22,6 %.

Dagegen ist der Abfall des Kristallisationsgrades mit kürzer werdender Kühlzeit bei den Proben mit Nukleierungsmittel wesentlich geringer.

So kann - durch den Einsatz des erfindungsgemäßen Nukleierungsmittels - bei einer Kühlzeit von 60 s - ein Kristallisationsgrad von 45,5 % gegenüber der entsprechenden Probe ohne Zusatz von Nukleierungsmittel (mit einem Kristallisationsgrad von nur 22.6 %) erreicht werden.

## Morphologische Untersuchungen

Auch die Gefügestruktur teilkristalliner Kunststoffe wird durch den Einsatz von Nukleierungsmitteln stark verändert. Zur genaueren Analyse der Wirkung des erfindungsgemäßen Nukleierungsmittels auf die Gefügestruktur wurden die folgenden Untersuchungen durchgeführt.

### 1. Polarisations-Mikroskopie

Für die Polarisations-Mikroskopie wurden Dünnschnitte der jeweiligen Poly-L-lactid-Probe von 10 µm analysiert.

Die Polarisations-Mikroskopie liefert für zwei Proben, die mit gleicher Kühlzeit von 60 s hergestellt wurden folgende Ergebnisse:

a) Diejenige Probe, die ohne Nukleierungsmittel hergestellt wurde, läßt nur wenige, kleine Sphärolithe erkennen, die in großen Bereichen mit amorphem Erscheinungsbild liegen.

b) Demgegenüber zeigt diejenige Probe, die mit einem Nukleierungsmittel hergestellt wurde ein wesentlich feinsphärolithischeres Gefüge. Die einzelnen Sphärolithe sind so dicht aneinandergerückt, daß amorphe Bereiche nicht mehr zu erkennen sind, wodurch ein - im Vergleich zur nicht-nukleierten Probe - hoher Kristallisationsgrad erreicht wird.

### 2. Lichtkleinwinkelstreuung

Mit Hilfe der Lichtkleinwinkelstreuung läßt sich der mittlere Sphärolithdurchmesser einer teilkristallinen Probe bestimmen.

Als Proben werden die für die Polarisations-Mikroskopie hergestellten Dünnschnitte eingesetzt. Die Lichtkleinwinkelstreuung liefert einen Beleg für den wesentlich kleineren Sphärolithdurchmesser bei den Proben, die mit dem erfindungsgemäßen Nukleierungsmittel behandelt wurden. Eine Abnahme der Sphärolithdurchmesser mit sinkender Kühlzeit ist bei Proben, die sowohl mit als auch ohne Nukleierungsmittel hergestellt wurden zu erkennen. Bei den Proben ohne Nukleierungsmittel ist die Abnahme jedoch deutlich größer.

In Fig. 5 ist die graphische Darstellung der Funktion des mittleren Sphärolithdurchmessers in Abhängigkeit von der Kühlzeit wiedergegeben.

Mechanische Untersuchungen

Der Einfluß der beschriebenen Werkstoffänderungen, die aus der Hinzufügung des erfindungsgemäßen Nukleierungsmittels resultieren, auf die physikalischen Charakteristika der daraus hergestellten Formteile wurden anhand folgender mechanischer Untersuchungen überprüft:

3-Punkt-Biegeversuch

Der 3-Punkt-Biegeversuch erfolgte mit einem gemäß DIN 53452 bzw. DIN 53457 [Deutsche Industrie Norm, Prüfungen von Kunststoffen, Bestimmung des Elastizitätsmoduls (E-Modul) im Zug-, Druck- und Biegeversuch]. Die Prüfdaten wurden bei einer Temperatur von 23° C und mit einer Prüfgeschwindigkeit von 5 mm/s ermittelt.

Es wurden Probenkörper folgender Abmessungen eingesetzt:
Länge: 25 mm
Verhältnis Länge/Höhe: 12.5
Stützweite: 20 mm

Mit dem 3-Punkt-Biegeversuch wurden E-Modul und Biegefestigkeit ermittelt. Dabei konnte eine deutliche Verbesserung der mechanischen Eigenschaften des nukleierten Poly-L-lactids gegenüber dem nicht-nukleierten Material festgestellt werden.

Fig. 6 zeigt eine graphische Darstellung der Funktion des E-Moduls in Abhängigkeit von der Kühlzeit.

Fig. 7 zeigt eine graphische Darstellung der Biegefestigkeit ebenfalls in Abhängigkeit von der Kühlzeit.

Die in Fig. 6 und Fig. 7 erkennbaren Steigerungen sind auf die - in den vorangegangenen Untersuchungen nachgewiesenen - Änderungen des Kristallisationsgrades und der Morphologie durch den Einsatz des erfindungsgemäßen Nukleierungsmittels zurückzuführen.

Die Ergebnisse - in ihrer Gesamtheit gewertet - zeigen, daß die größten Änderungen des Kristallisationsgrades bei geringen Abkühlzeiten zu erkennen sind. Wie Fig. 8 - in der die prozentualen Änderungen der gemessenen Größen von Formteilen mit Nukleierungsmittel gegenüber Formteilen ohne Nukleierungsmittel in Abhängigkeit von der Kühlzeit dargestellt sind - entnommen werden kann, kommt es - beispielsweise bei einer Kühlzeit von 60 s - zu einer Steigerung des Kristallisationsgrades um fast 120 %. Der erhöhte Kristallisationsgrad geht mit einer Abnahme der amorphen Bereiche zwischen den einzelnen Sphärolithen einher. Da diese amorphen Bereiche bei einer mechanischen Belastung wie Fehlstellen wirken, die die Kräfte nur unzureichend übertragen können, führt eine Verringerung der amorphen Zwischenräume zu einer Erhöhung der Steifigkeit.

Der E-Modul teilkristalliner Kunststoffe in Abhängigkeit vom Kristallisationsgrad läßt sich mathematisch erfassen [Deutsche Industrie Norm, Prüfung von Kunststoffen, Bestimmung des Elastizitätsmoduls im Zug-, Druck- und Biegeversuch], wobei allgemein festgestellt werden kann, daß der E-Modul mit wachsendem Kristallisationsgrad steigt. Bei den untersuchten Formteilen konnte eine Steigerung des E-Moduls bei einer Abkühlzeit von 60 s um 40 % festgestellt werden.

Die Erhöhung der prozentualen Änderung des Kristallisationsgrades und die damit verbundene Abnahme der Änderung des Sphärolithdurchmessers durch den Einsatz des erfindungsgemäßen Nukleierungsmittels führen zu einer nahezu konstanten Erhöhung des E-Moduls über dem gesamten untersuchten Kühlzeitbereich.

Versuche unterhalb einer Kühlzeit von 60 s, konnten nur mit nukleierten Proben durchgeführt werden. Der Kristallisationsgrad fällt zwar bei einer Abkühlzeit unterhalb von 50 s stark ab, dennoch liegt er bei einer Abkühlzeit von 30 s noch bei 33 % (vgl. Fig. 4). Der mittlere Sphärolithdurchmesser bleibt unterhalb von 60 s nahezu konstant. Dies führt zwar zu einem Abfall des E-Moduls und der Biegefestigkeit, beide Größen liegen jedoch bei 30 s immer noch deutlich über den Werten, die für die nicht-nukleierten Proben bei einer Kühlzeit von 60 s ermittelt wurden.

Formteile mit guten mechanischen Eigenschaften und einem Kristallisationsgrad von 46 %, konnten durch den Einsatz des erfindungsgemäßen Nukleierungsmittels, bei einer relativ kurzen Kühlzeit von 50 s, hergestellt werden.

Zusammenfassend kann festgestellt werden, daß sich durch den Zusatz des erfindungsgemäßen Nukleierungsmittels Formteile mit guten mechanischen Eigenschaften und einem hohen Kristallisationsgrad, bei kleinen Kühlzeiten herstellen lassen.

Somit ergibt sich durch den Einsatz von Polyglycolsäure als Nukleierungsmittel eine deutliche Kühlzeitverkürzung und damit eine Reduzierung der Zykluszeit.

**Patentansprüche**

1. Verwendung von Polyglycolsäure und/oder ihrer Derivate gemäß der allgemeinen Formel I

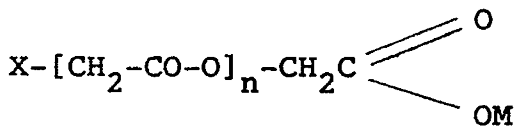

(I)

- worin
  X Wasserstoff, ein Halogenatom oder eine Hydroxylgruppe;
  M ein Metall-Kation oder Wasserstoff und
  n eine ganze Zahl >20
  bedeutet -
  als Nukleierungsmittel.

2. Verwendung von Polyglycolsäure und/oder ihrer Derivate gemäß der allgemeinen Formel I nach Anspruch 1, dadurch gekennzeichnet, daß n eine ganze Zahl im Bereich zwischen 20 und 300 bedeutet.

3. Verwendung von Polyglycolsäure und/oder ihrer Derivate gemäß der allgemeinen Formel I nach Anspruch 1, dadurch gekennzeichnet, daß n eine ganze Zahl im Bereich zwischen 50 und 150 bedeutet.

4. Verwendung vcn Polyglycolsäure und/oder ihrer Derivate gemäß der allgemeinen Formel I nach einem der Ansprüche 1 bis 3, daß X - bezogen auf das gesamte Polymerisat - partiell Chlor bedeutet.

5. Ausführungsform nach Anspruch 4, dadurch gekennzeichnet, daß der Gehalt an kovalent gebundenem Chlor zwischen 0 und 2 Gew.-% liegt.

6. Ausführungsform nach Anspruch 4, dadurch gekennzeichnet, daß der Gehalt an kovalent gebundenem Chlor zwischen 0 und 1 Gew.-% liegt.

7. Ausführungsform nach Anspruch 4, dadurch gekennzeichnet, daß der Gehalt an kovalent gebundenem Chlor zwischen 0 und 0,6 Gew-% liegt.

8. Verwendung von Polyglycolsäure und ihrer Derviate gemäß der allgemeinen Formel I nach einem der Anspruche 1 bis 7, dadurch gekennzeichnet, daß M - bezogen auf das gesamte Polymere - partiell Natrium bedeutet.

9. Ausführungsform nach Anspruch 8, dadurch gekennzeichnet, daß der Gehalt an Natrium zwischen 0 und 5 Gew.-% liegt.

10. Ausführungsform nach Anspruch 8, dadurch gekennzeichnet, daß der Gehalt an Natrium zwischen 0 und 2 Gew.-% beträgt.

11. Ausführungsform nach Anspruch 8, dadurch gekennzeichnet, daß der Natriumgehalt zwischen 0 und 1 Gew.-% beträgt.

12. Verwendung von Polyglycolsäure und/oder ihrer Derivate gemäß der allgemeinen Formel I nach einem der Ansprüche 1 bis 11, dadurch gekennzeichnet, daß der Gehalt an ionogen gebundenem Chlorid im Bereich zwischen 0 und 5 Gew.-% liegt.

13. Ausführungsform nach Anspruch 12, dadurch gekennzeichnet, daß der Gehalt an ionogen gebundenem

Chlorid zwischen 0 und 1 Gew.-% liegt.

**14.** Verwendung von Polyglycolsäure und/oder ihren Derivaten gemäß der allgemeinen Formel I nach einem der Ansprüche 1 bis 13 zur Nukleierung von biologisch abbaubaren Polymeren.

**15.** Verwendung von Polyglycolsäure und/oder ihren Derivaten gemäß der allgemeinen Formel I nach Anspruch 14 zur Nukleierung von biologisch abbaubaren Polyestern.

**16.** Verwendung von Polyglycolsäure und/oder ihren Derivaten gemäß der allgemeinen Formel I nach Anspruch 15 zur Nukleierung von biologisch abbaubaren Polyestern auf der Basis von L-Laktid, D, L-Laktid, meso-Laktid, Glycolid, Trimethylencarbonat, Dioxanon und/oder Caprolacton.

**17.** Verwendung von Polyglycolsäure und/oder ihren Derivaten gemäß der allgemeinen Formel I nach einem der Ansprüche 1 bis 13 zur Nukleierung von Polyethylen, Polypropylen, Polybutylen, Polyoxymethylen und Polyamiden.

**18.** Nucleierungsmittel, dadurch gekennzeichnet, daß es einen Gehalt an Polyglycolsäure und/oder ihrer Derivate gemäß einem der Ansprüche 1 bis 13 aufweist.

Fig.1

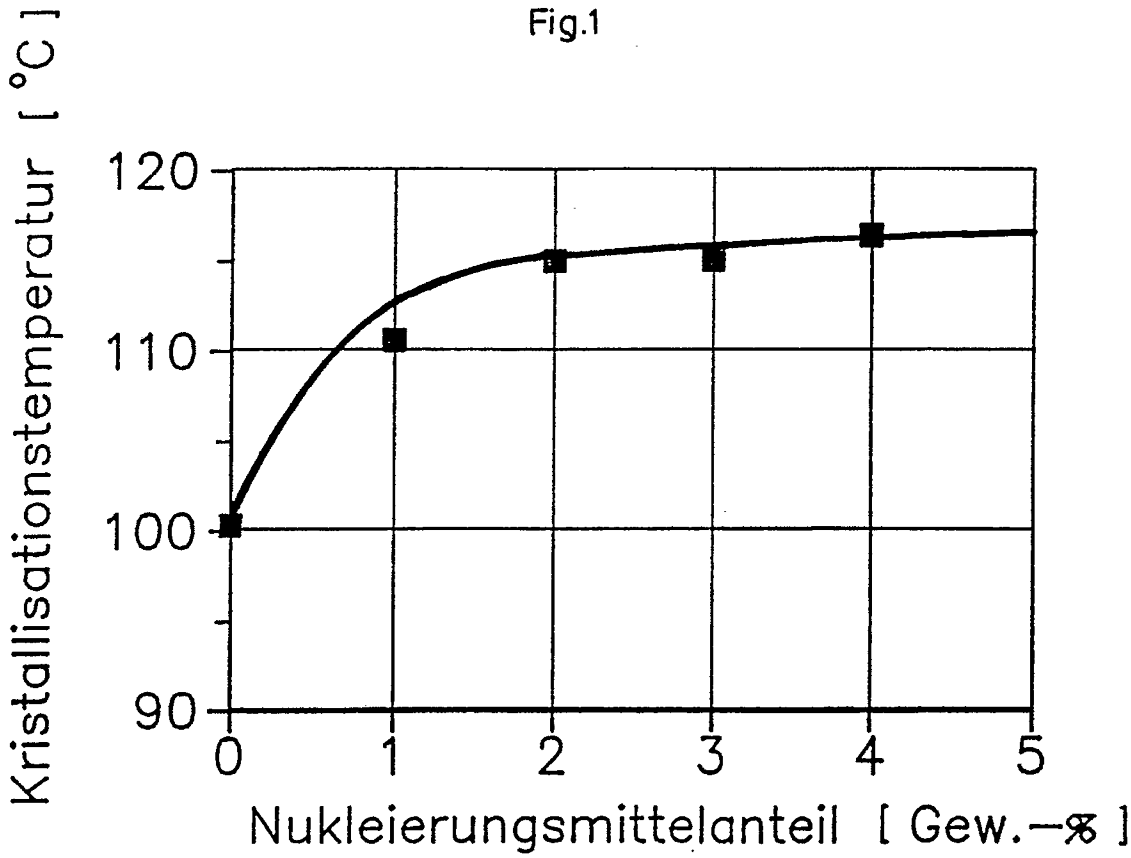
Kristallisationstemperatur [ °C ]
120
110
100
90
0
1
2
3
4
5
Nukleierungsmittelanteil [ Gew.-% ]

Fig. 2

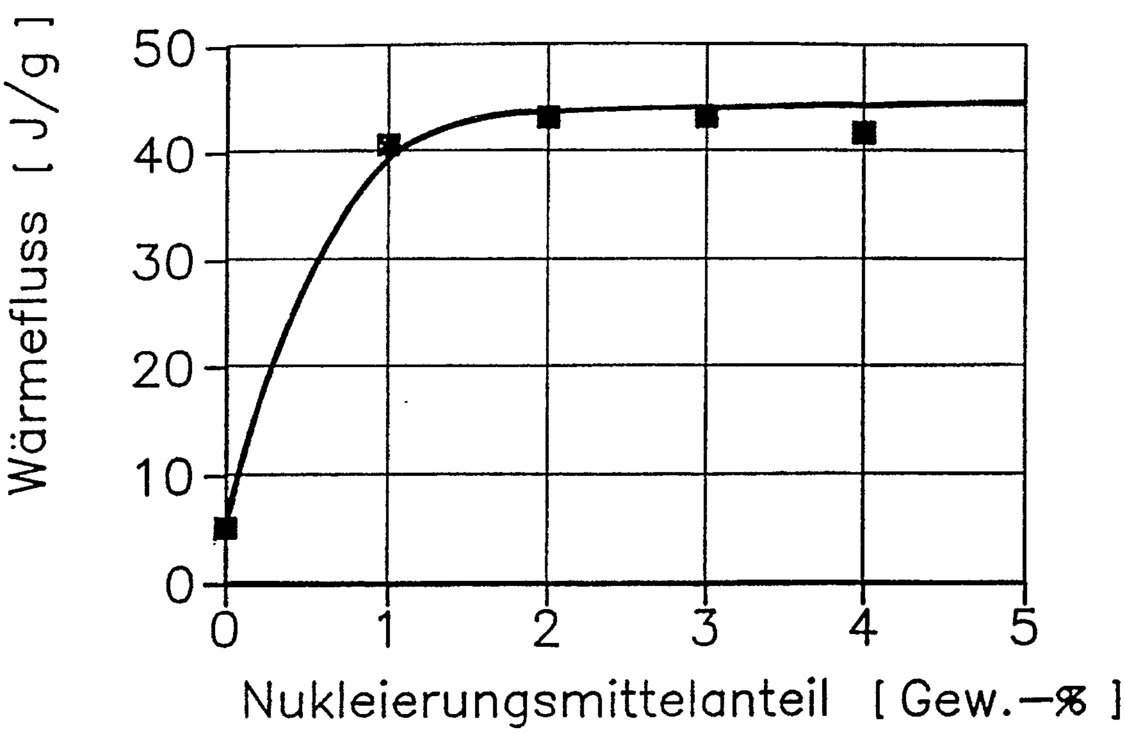
Wärmefluss [ J/g ]
50
40
30
20
10
0
0
1
2
3
4
5
Nukleierungsmittelanteil [ Gew.-% ]

Fig. 3

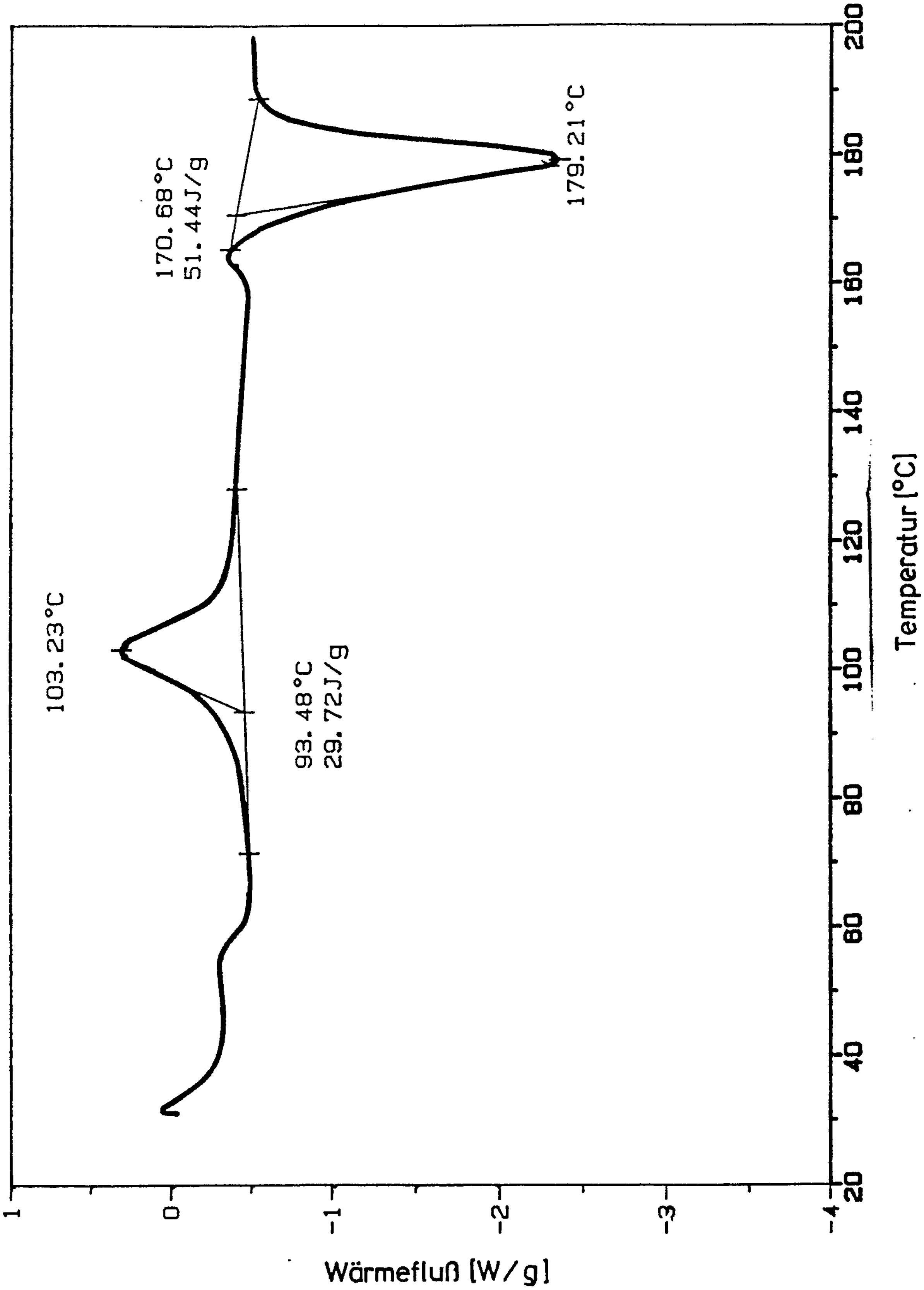
170.68°C
51.44J/g
179.21°C
103.23°C
93.48°C
29.72J/g
Temperatur [°C]
20
40
60
80
100
120
140
160
180
200
Wärmefluß [W/g]
1
0
-1
-2
-3
-4

Fig. 4

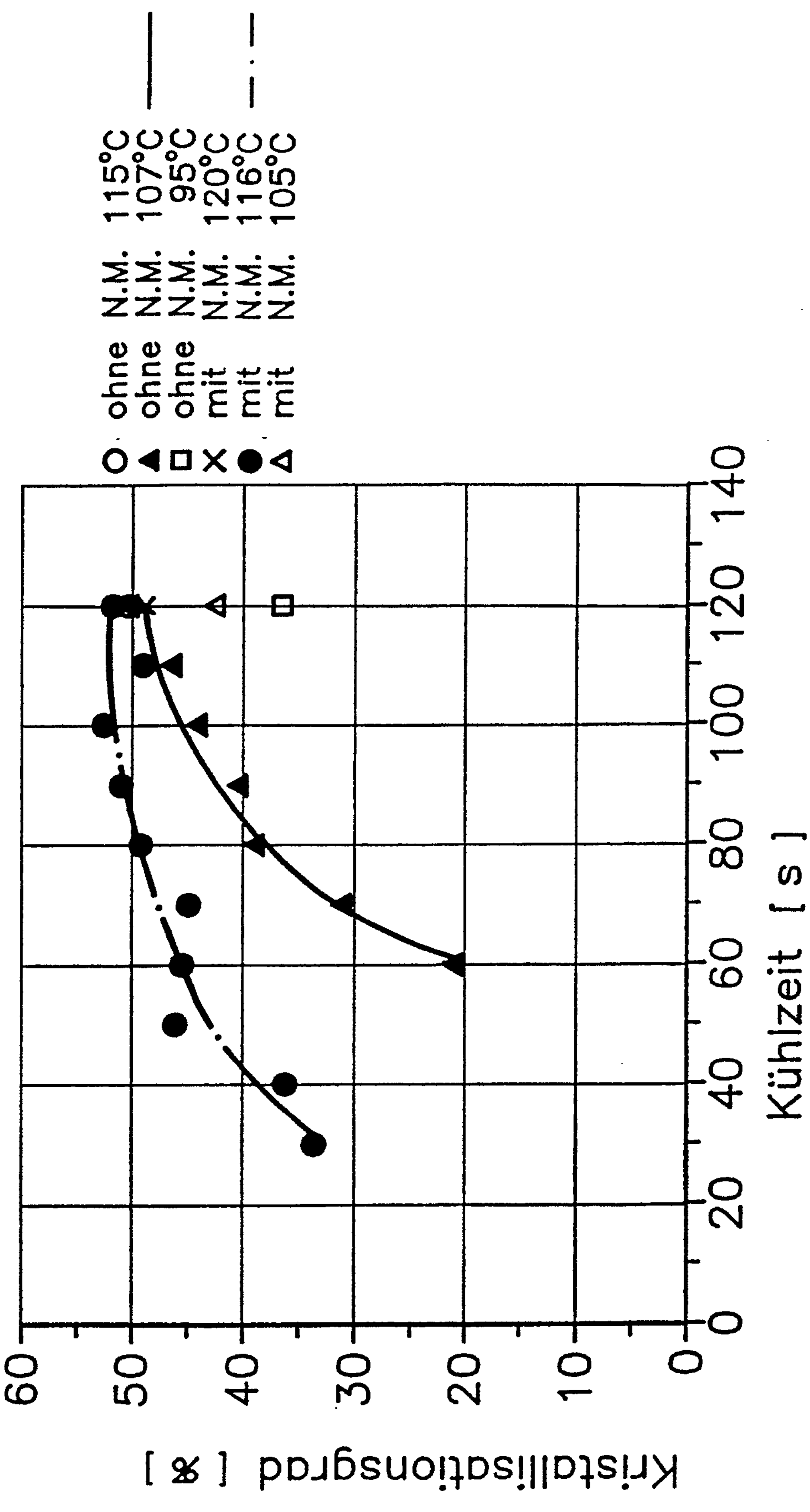

ohne N.M. 115°C
ohne N.M. 107°C
ohne N.M. 95°C
mit N.M. 120°C
mit N.M. 116°C
mit N.M. 105°C
Kristallisationsgrad [ % ]
0
10
20
30
40
50
60
Kühlzeit [ s ]
0
20
40
60
80
100
120
140

Fig.5

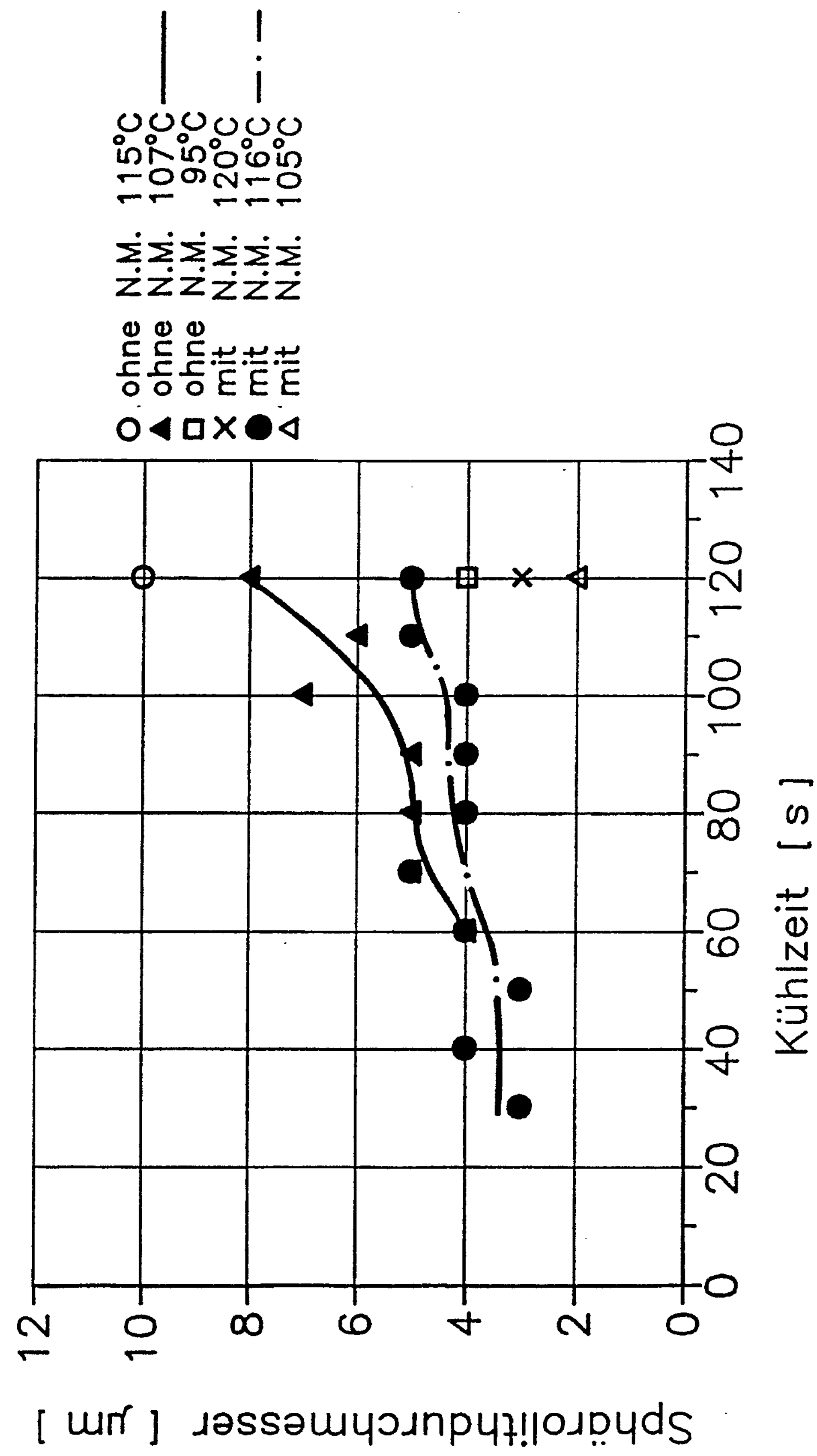
○ ohne N.M. 115°C
▲ ohne N.M. 107°C
□ ohne N.M. 95°C
× mit N.M. 120°C
● mit N.M. 116°C
△ mit N.M. 105°C
Sphärolithdurchmesser [ µm ]
12
10
8
6
4
2
0
0
20
40
60
80
100
120
140
Kühlzeit [ s ]

Fig.6

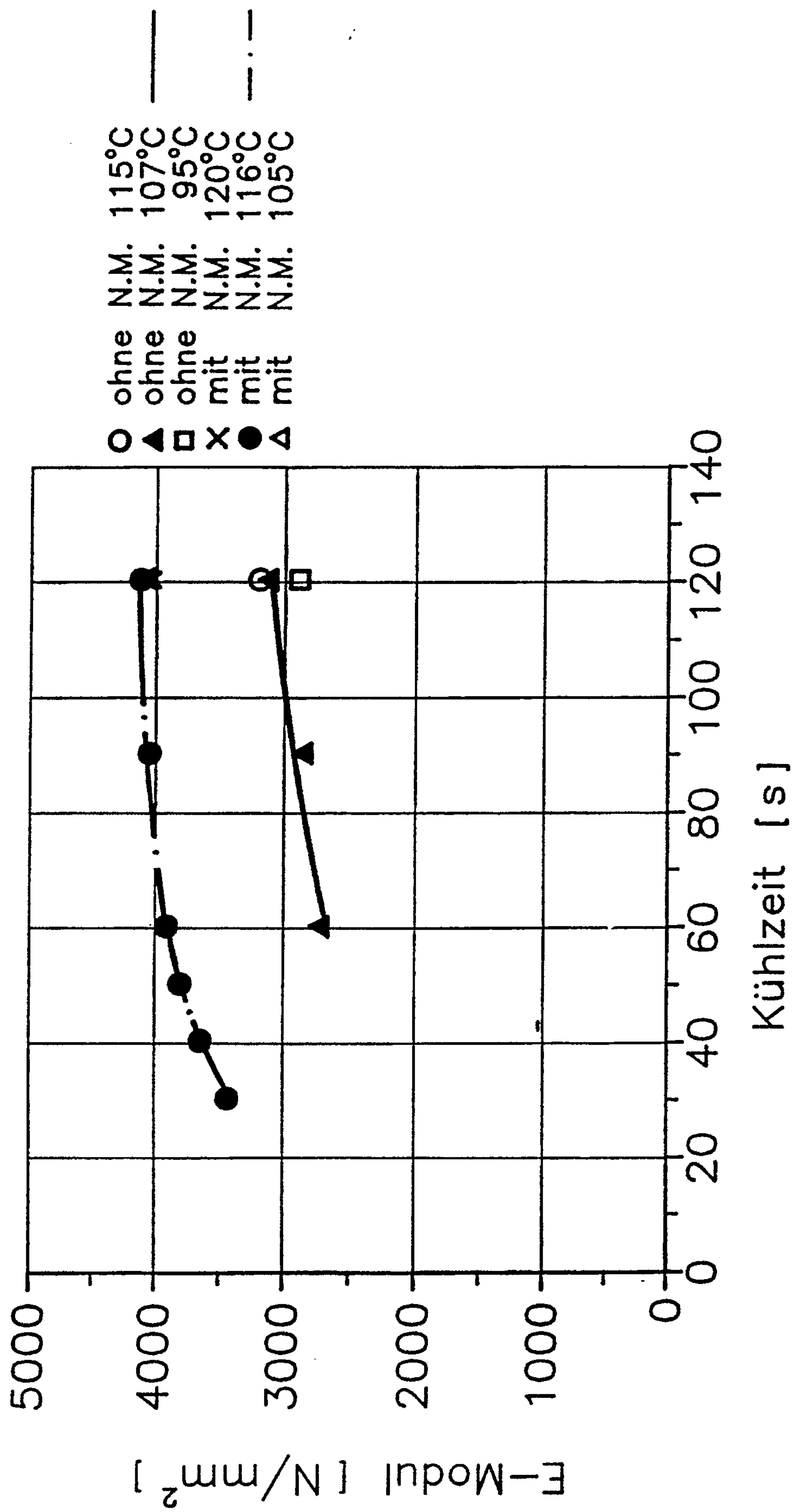
○ ohne N.M. 115°C
▲ ohne N.M. 107°C
□ ohne N.M. 95°C
× mit N.M. 120°C
● mit N.M. 116°C
△ mit N.M. 105°C
E–Modul [ N/mm² ]
5000
4000
3000
2000
1000
0
Kühlzeit [ s ]
0
20
40
60
80
100
120
140

Fig.7

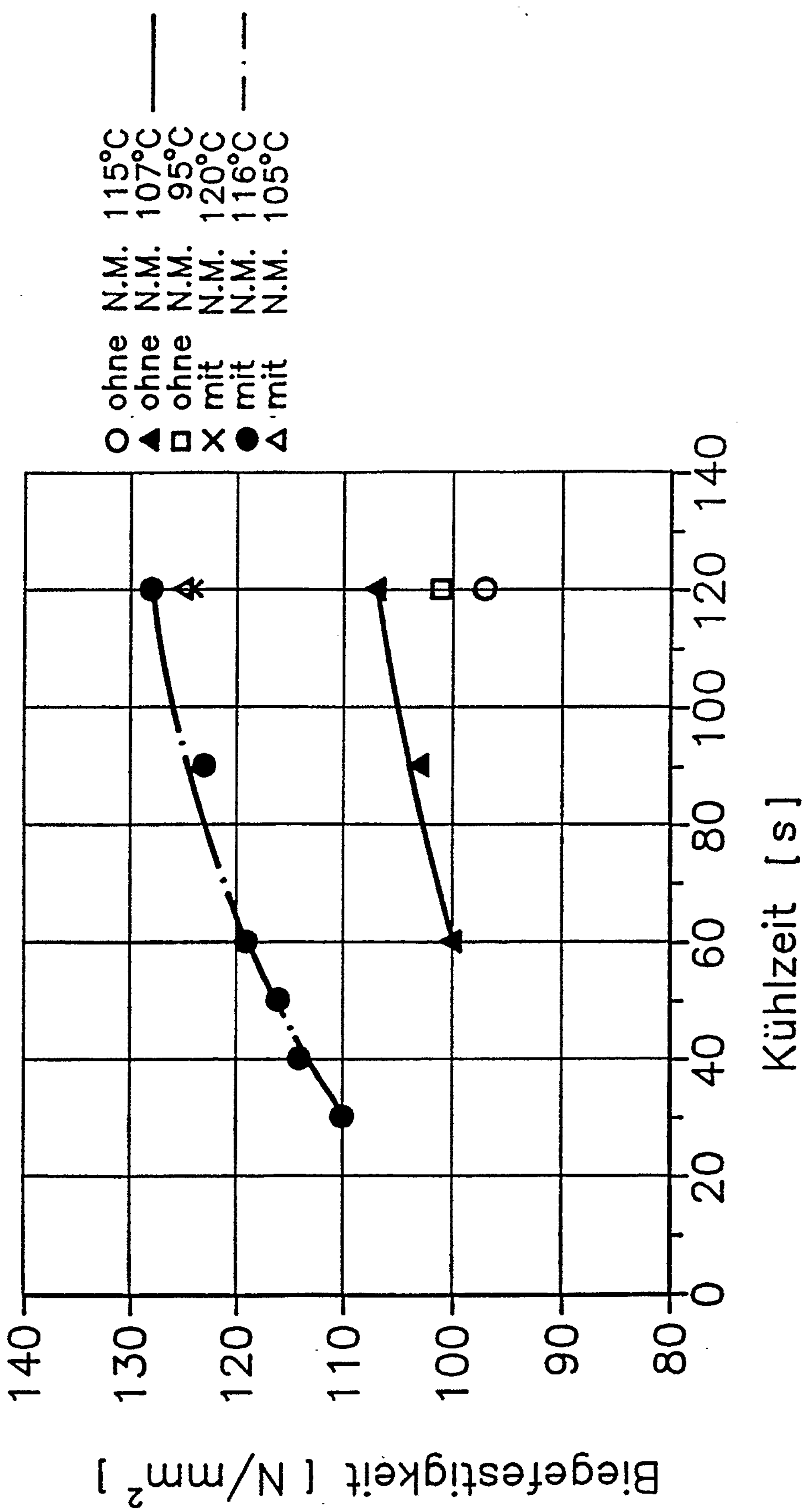
ohne N.M. 115°C
ohne N.M. 107°C
ohne N.M. 95°C
mit N.M. 120°C
mit N.M. 116°C
mit N.M. 105°C
Biegefestigkeit [ N/mm² ]
140
130
120
110
100
90
80
0
20
40
60
80
100
120
140
Kühlzeit [ s ]

Fig.8

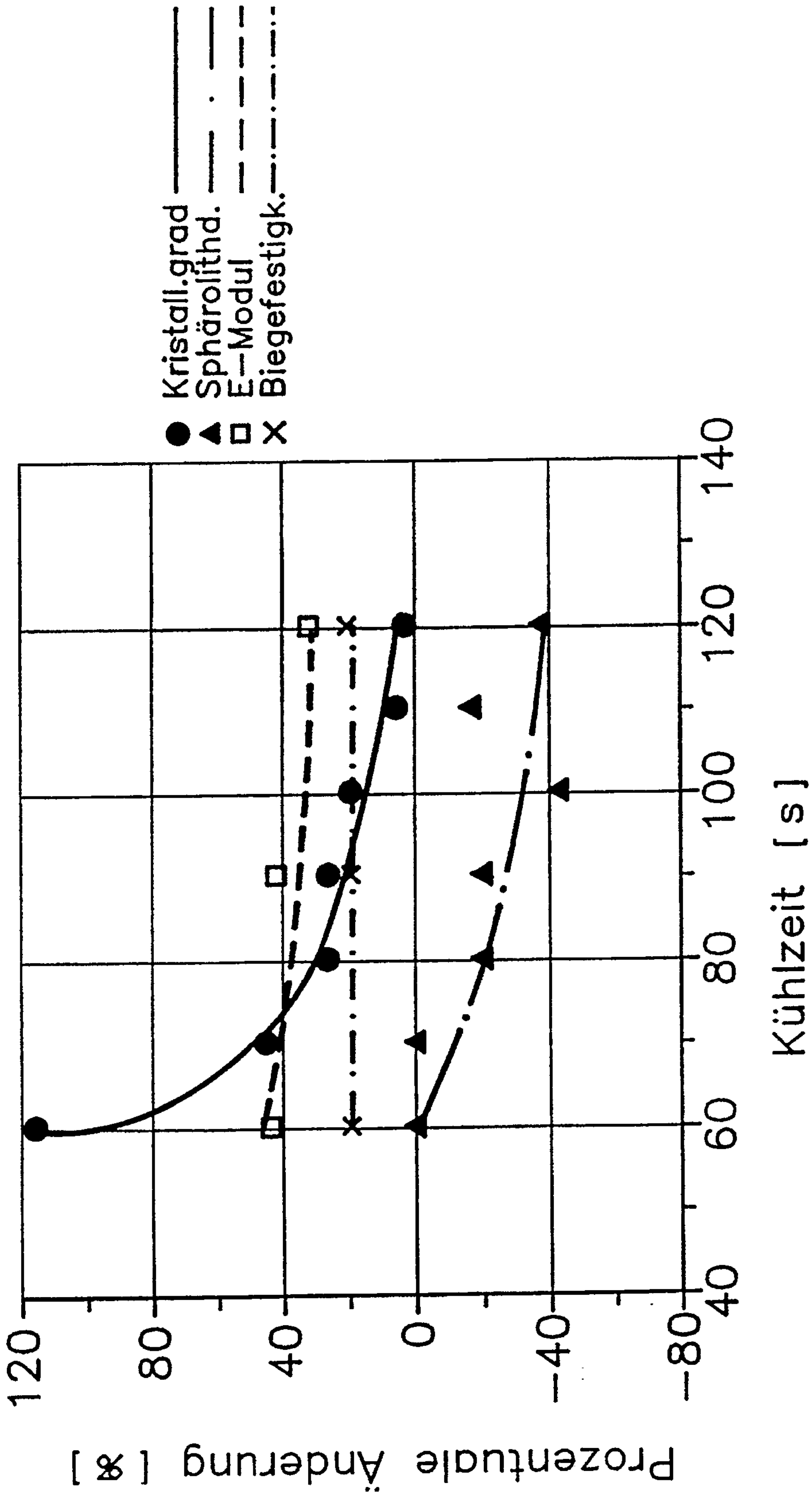
Kristall.grad
Sphärolithd.
E—Modul
Biegefestigk.
Kühlzeit [s]
Prozentuale Änderung [%]
40
60
80
100
120
140
120
80
40
0
−40
−80